# EUROPEAN PATENT APPLICATION

(11) **EP 3 536 330 A1**
(43) Date of publication of application: **11.09.2019**
(21) Application number: 18160465.3
(22) Date of filing: 07.03.2018
(51) Int. Cl.: A61K 35/76, A61P 3/02

(54) **INFANT & TODDLER FORMULA CONTAINING A VIRUS PREPARATION**

(71) Applicant: Jennewein, Stefan, 53604 Bad Honnef (DE)
(72) Inventor: Jennewein, Stefan, 53604 Bad Honnef (DE)
(74) Representative: Witte, Weller & Partner Patentanwälte mbB

(57) **Abstract**

Disclosed are infant or toddler formulae containing a lytic virus preparation for the modification of an infant's gastrointestinal microbiota, and for the protection against infectious diseases.

## Description

The present invention relates to infant formula or toddler nutrition product. More specifically, the present invention concerns infant formula and toddler nutrition product for modulating the gut microbiota of an infant or toddler. The invention relates to a nutritional product containing a virus specific for fighting a human pathogen either for protection (for prophylactic purposes), or to fight a certain microbial infection or dysbiosis.

### Background

Gastrointestinal infections of pre-term infants, neonates, infants and toddlers are one of the major causes of infant and toddler casualties in the world. Enteropathogenic infections are particularly severe for these children due to their hardly developed immune system.

Examples of enteropathogenic bacteria include certain species o r strains of *Escherichia coli, Klebsiella, Clostridium perfringens, Staphylococcus epidermidis, Shigella, Yersinia, Salmonella, Vibrio cholerae* and *Campylobacter jejuni.* An example for an eukaryotic pathogen causing gastrointestinal infections is *Cryptosporidium (Cryptosporidium hominis, Cryptosporidium parvum),* causing watery diarrhea (intestinal cryptosporidiosis). In addition to lactose, lipids, proteins, minerals and complex oligosaccharides, human milk also contains nucleic acid molecules. Some of these nucleic acid molecules code for viruses. Most of these viruses are not pathogenic to humans. The inventor believes that these viruses contribute to the health of a breast fed infant by fighting pathogens and/or modulating the microbiome of the infant in that these viruses comprise bacteriophages which target and lyse pathogenic or anyhow undesired bacteria in the infant's gut and/or to promote growth and enhance the number of desired bacteria in the infant's gut. For example, Neonatal Necrotizing Enterocolitis (NEC) is a common life-threatening emergency of the gastrointestinal tract in newborns which could benefit from the inclusion of one or more bacteriophages fighting microorganism such as, but not limited to, certain *Escherichia coli, Klebsiella, Clostridium perfringens, Clostridium difficile, Vibrio cholerae* and/or *Staphylococcus epidermidis* strains. Examples of desired bacterial strains in the infant's gut are some *Bifidobacterium* strains such as *Bifidobacterium longum* ssp. *infantis, Bifidobacteriudm breve, Bifidobacterium bifidum,* and some *Lactobacillus* strains *(Lactobacillus johnsonii, Lactobacillus grasseri, Lactobacillus paracasei, Lactobacillus casei, Lactobacillus rhamnosus; Lactobacillus plantarum).*

Infant formula and food for toddlers that are commercially available today do not deliberately contain viruses such as bacteriophages to modify an infant's or toddler's microbiome. Also todays infant or toddler nutritional products do not contain viruses for preventing or even cure the infant from enteropathogenic infections (by bacterial or eukaryotic pathogens).

It has therefore been an object to utilize viruses for the modification of the gut microbiota of infants and toddlers. In another objective lytic viruses are added to protect infant and toddler from certain pathogenic microorganisms.

The object is solved by providing an infant formula which contains a lytic virus preparation, said lytic virus preparation is lytic to one or more undesired microbial species or strains within the infant's gastrointestinal tract.

Another aspect of relevance is the application of a nutritional formula containing virus for the manipulation of the neonate or toddlers microbiome. Thus applying a virus or a cocktail of several different viruses for decreasing the presence of a certain microbial strain or several microbial strains in a microbiome to the benefit of other microbial strains (microbiome modulation).

This microbiome modulation can be re-enforced by including into the virus containing formula (e.g. a powder infant formula, a liquid infant and toddler nutrition product, milk drink) certain substances (e.g. prebiotics), which promote the growth of desirable microorganisms. Even more beneficial would be the combination of both prebiotic substances and at least one probiotic strain in a product containing a virus preparation. Most beneficial would be the combination of both prebiotic substances and beneficial/probiotic microbial stains in a virus containing formula containing several different viruses (or a formula containing a cocktail of viruses). Thus, by this means early life dysbiosis can be prevented or corrected.

### Summary

In a first aspect, provided is an infant or toddler formula containing one or several lytic viruses specific for a human microbial pathogen (e.g. a bacteriophage specific for a human bacterial pathogen, such as a *Campylobacter jejunii* bacteriophage or a phage specific for a enteropathogenic *E. coli* strain). Thus the infant or toddler formula should protect against the infection of certain microbial pathogens.

In a second aspect, provided is an infant or toddler formula for modulating an infant's gastrointestinal microbiota (e.g. in a condition also referred to as dysbiosis), wherein said formula contains a lytic virus preparation, said virus preparation is lytic to selected undesired microorganisms of an infant's or toddler's microbiota for producing lysis of the selected microorganisms in the gastrointestinal tract.

In a third aspect, provided is the use an infant formula which contains a lytic virus preparation, wherein said virus preparation is lytic to selected undesired microorganisms of an infant's microbiota for the modulation of the infant's gastrointestinal microbiota.

In a fourth aspect, provided is a method for modulating an infant's gastrointestinal microbiota by lysis of selected undesired microorganism in the infant's gastrointestinal tract, wherein an infant formula is fed to the infant, wherein said infant formula contains a lytic virus preparation, said virus preparation being lytic to the undesired microorganisms.

In a fifth aspect, provided is an infant formula or toddler nutrition product for modulating an infant's or toddlers gastrointestinal microbiota, wherein said formula/product contains a lytic virus preparation and at least one beneficial microbial strain (probiotic strain). Said virus preparation is lytic to selected undesired microorganisms of an infant's microbiota for producing lysis of the selected microorganisms in the infant's gastrointestinal tract. In order to replenish the microbiota, after parts of the microbiota got reduced or eliminated from the microbiota, the formula provides probiotic a microorganism or several microorganisms to fill the available ecological niches in the microbiota. In a sixth aspect, provided is an infant or toddler formula for modulating and maintaining an infant's or toddlers gastrointestinal microbiota, wherein said formula contains a lytic virus preparation and at least one prebiotic compound (e.g. galactooliogsaccharides (GOS), fructooligosaccharides (FOS), or a combination of several human milk oligosaccharides) promoting the growth of certain microorganism or microorganisms.

In a seventh aspect, provide an infant formula or toddler nutrition product for maintaining and or modulating an infant's or toddlers gastrointestinal microbiota, wherein said formula contains a lytic virus preparation, said virus preparation being lytic to undesirable microorganisms, and at least one prebiotic substance promoting the growth of desirable microorganisms (e.g. one or more human milk oligosaccharides) and at least one probiotic strain.

We define an infant as a child of 0 to 12 months of age. A toddler is a child 12 to 60 months of age.

### Detailed description

According to the first aspect, provided is an infant formula or toddler nutrition product containing a lytic virus preparation. The virus preparation is lytic to selected microorganisms of an infant's or toddler's microbiota for producing lysis of the selected microorganisms in the infant's gastrointestinal tract. Said selected miroorganisms are one or more species or strains of undesired microorganism. Said undesired microorganisms may be pathogenic microorganism such as - for example - enteropathogenic bacteria. Additionally and/or alternatively, said undesired microorgnisms may be non-pathogenic microorganisms of the gastrointestinal microbiota.

The terms "microbiota" and "microbiome" as used herein, refer to an ecological community of commensal, symbiotic and pathogenic microorganism found in the gastrointestinal tract of a mammalian subject. A typical microbiota includes bacteria, archeae, protists, fungi and viruses. Wherein "microbiota" more specifically refers to the microorganisms, the synonymous term "microbiome" refers to the collective genomes of the microorganisms that reside in the subject's gastrointestinal tract.

Dysbiosis refers to a perceived imbalance in the proportions of bacterial groups making up a microbial community. Conditions like obesity (overweight), infantile colic, necrotizing enterocolitis can be a result of dysbiosis. Possible causes of dysbiosis are the use of antibiotics (e.g. broad spectrum β-lactam antibiotics), mode of delivery (e.g. cesarean section), maternal dysbiosis, infant formula, maternal antibiotic use etc.

In an additional and/or alternative embodiment, said lytic virus preparation of the infant formula comprises at least one bacteriophage or prophage, said at least one prophage being preferably present as extrachromosomal plasmid form or integrated into the genome of a microorganism. It is understood that said at least one bacteriophage or prophage is a lytic bacteriophage or prophages respectively, and thus represents the lytic virus of the lytic virus preparation. A bacteriophage is a virus that infects and replicates in bacteria and archeae. Bacteriophages comprise proteins that encapsulate the DNA genome or the RNA genome of the bacteriophage. They replicate within a bacterial cell following the injection of their genome into the bacterial cytoplasm. Bacteriophages are ubiquitus in the biosphere. A prophage is a bacteriophage genome. It may exist inserted and integrated into the bacterial chromosome or as an extrachromosomal plasmid. The prophage represents a latent form of its bacteriophage and does not cause disruption of the bacterial cell. The prophage within a bacterial cell may be induced to begin viral replication and the lytic cycle.

In an additional and/or alternative embodiment, the at least one prophage is present as extrachromosomal plasmid form or is present integrated into the genome of a microorganism. In an additional and/or alternative embodiment, the microorganism containing the prophage is constituent of the infant formula. Providing the lytic virus as prophage is advantageous if the virus as such is sensitive to an acidic environment such as present in the gastric juice of the subject's stomach. Under suitable conditions, the prophage within the microorganism becomes lytic in the gastrointestinal tract, i.e. in the gut, small intestine, large intestine, cecum, colon and/or rectum.

In an additional and/or alternative embodiment, the virus of the preparation or the lytic virus preparation targets a microorganism of the infant's or toddler's microbiota. Preferably, the virus or lytic virus targets the microorganism(s) overgrowing. The microorganism(s) that is/are undesired are lysed by the lytic virus of the lytic virus preparation upon feeding the infant or toddler formula, such that the number of said undesired microorganisms in the gastrointestinal tract of the infant or toddler is reduced and its microbiota is modified, i.e. normalized. Normalization of the infant's or toddler's microbiota by lysing one or more of the undesired microbial species not only alleviates symptoms of syndromes that are associated with a prevalence of the undesired microorganisms in the infant's gastrointestinal tract. It is known that specific bacteriophage strains only infect a narrow range of hosts. This specificity renders bacteriophages safe for use in humans and animals, and are unlikely to cause undesired side effects. In addition, this specificity permits a specific and precise modulation of an infant's microbiota in that only one or a few microbial species or strains are lysed upon feeding of the infant formula to an infant in need thereof. Other than with antibiotics, it is possible to specifically target the undesired bacterial species without affecting other species of the microbiota. Without wishing to be bound by any theory, it is believed that the lysis of the undesired microorganism(s) improves the composition of the infant's or toddler's microbiota, which then more closely resembles the original and natural composition.

In an additional and/or alternative embodiment, the lytic virus or lytic virus preparation targets a microorganism selected from the group consisting of gram positive and/or gram negative bacteria, preferably a microorganism selected from one of the families selected from the group consisting of Alcaligenaceae, Bacteroidaceae, Caulobacteraceae, Christenssenellaceae, Clostridiaceae , Coriobacteriaceae, Enterobacteriaceae, Enterococcaceae, Erysipelotrichaceae, Halomonadaceae, Helicobacteraceae, Lachnospiraceae, Peptostreptococcaceae, Phyllobacteriaceae, Porphyromonadaceae, Prevotellaceae, Rikenellaceae, Ruminococcaceae, Streptococcaceae, Veillonellaceae, Vibrionaceae, Verrucomicrobiaceae.

In an additional and/or alternative embodiment, the at least one bacteriophage or prophage targets a microorganism selected from one of the genera selected from the group consisting of *Bacillus, Bacterioides, Clostridium, Cryptococcus, Enterobacter, Enterococcus, Escherichia, Haemophilus, Helicobacter, Klebsiella, Listeria, Prevotella, Proteus, Pseudomonas, Salmonella, Shigella and Staphylococcus, Streptococcus, Rothia, Staphylococcus, Clostridium, Escherichia, Shigella, Salmonella, Campylobacter, Chloerae, Vibrio* and *Yersinia.*

In an additional and/or alternative embodiment, the at least one *bacteriophage or prophage targets a microorganism selected from the group consisting of Bacteroidetes bacterium, Bacteroides nordii, Bacteroides fragilis, Caminicella sporogenes, Campylobacter jejuni, Capnocytophaga ochracea, Clostridium difficile, Dysgonomonas gadei, Enterobacter cloacae, Escherichia coli, Eubacterium sp. Helicobacter acinonachis, Helicobacter anseris, Helicobacter aurati, Helicobacter bilis, Helicobacter bizzozeronii, Helicobacter brantae, Helicobacter canadensis, Helicobacter canis, Helicobacter cetorum, Helicobacter cholecystus, Helicobacter cinaedi, Helicobacter cynogastris, Helicobacter felis, Helicobacter fenelliae, Helicobacter ganmani, Helicbacter hepaticus, Helicobacter mesocricetorum, Helicobacter marmotae, Helicobacter muridarum, Helicobacter mustelae, Helicobacter pametensis, Helicobacter pullorum, Helicobacter pylori, Helicobacter rappini, Helicobacter rodentium, Helicobacter salomonis, Helicobacter trogontum, Helicobacter typhlonius, Helicobacter winghamensis, Nubsella zeaxanthinifaciens, Parabacterioides merdae, Parabacteroides goldsteinii, Pedobacter koreensis, Petrimonas sulfuriphila, Porphyromonas catoniae, Porphyromonase sp., Prevotellacease bacterium, Proteiniphilum acetatigenes, Riemerella nanatipstifer, Salmonella enterica, Salonella bongori, Salmonella subterranean, Salamonella typhimurium, Shigella dysenteriae, Shigella flexneri, Shigella boydii, Shigella sonnei, Spingobacterium sp., Staphylococcus aureus, Vibrio cholerae, Yersinia enterocolitica, Yersinia pseudotuberculosis* and *Yersinia pestis.*

In an additional and/or alternative embodiment, the bacteriophage or prophage is selected from the order of Caudavirales; Microviridae, Corticoviridae, Tectiviridae, Leviviridae, Cystoviridae, Inoviridae, Lipothrixviridae, Rudiviridae, Plasmaviridae, Fuselloviridae or Ligamenvirales, preferably but not limited from the family of Myoviridae, Siphoviridae,Podoviridae and Inovirus..

The bacteriophage or prophage may be a naturally occurring bacteriopahge or may be a genetically-engineered bacteriophage.

In an additional and/or embodiment, the virus is present in the infant formula or toddler nutrition product in an amount of at least 1 pfu/g, at least 10² pfu/g, at least 10³ pfu/g, at least 10⁴ pfu/g, at least 10⁵ pfu/g, at least 10⁶ pfu/g, at least 107 pfu/g, at least 10⁸ pfu/g, at least 10⁹ pfu/g, at least 10¹⁰ pfu/g, at least 10¹¹ pfu/g, at least 10¹² pfu/g, at least 10¹³ pfu/g, at least 10¹⁴ pfu/g, at least 10¹⁵ pfu/g, at least 10¹⁶ pfu/g, at least 10¹⁷ pfu/g, at least 10¹⁸ pfu/g or at least 10¹⁹ pfu/g, and optionally does not exceed 10²⁰ pfu/g.

In an additional and/or embodiment, the virus is present in a liquid infant formula or liquid toddler nutrition product in an amount of at least 0.001 pfu/ml, at least 0.1 pfu/ml, at least 1 pfu/ml at least 10¹ pfu/ml, at least 10² pfu/ml, at least 10³ pfu/ml, at least 10⁴ pfu/ml, at least 10⁵ pfu/ml, at least 10⁶ pfu/ml, at least 10⁷ pfu/ml, at least 10⁸ pfu/ml, at least 10⁹ pfu/ml, at least 10¹⁰ pfu/ml, at least 10¹¹ pfu/ml, at least 10¹² pfu/ml, at least 10¹³ pfu/ml, at least 10¹⁴ pfu/ml, at least 10¹⁵ pfu/ml, at least 10¹⁶ pfu/ml, at least 10¹⁷ pfu/ml, at least 10¹⁸ pfu/ml or at least 10¹⁹ pfu/ml, and optionally does not exceed 10²⁰ pfu/ml.

In an additional and/or alternative embodiment, the infant formula or toddler nutritional product further contains at least one probiotic microorganism. Said at least one probiotic microorgansim is a desired microorganism in the infant's or toddler's gut, and is not a target of the virus within the infant formula or toddler nutrition product. Said at least one probiotic mircroorganism is selected from the microorganisms that are underrepresented in the microbiota of the infant or toddler. Said at least one probiotic microorganism is intended to improve the modification of the infant's or toddler's microbiota in that said at least one probiotic microorganism supplements the number of desired microbial species in the microbiome.

In an additional and/or alternative embodiment, the at least one probiotic microorganism is selected from the group consisting of *Bacillus coagulans, Lactobacillus acidophilus, Lactobacillus plantarum, Lactobacillus casei subsp. Ccasei, Lactobacillus casei subsp. rhamnosus, Lactobacillus zeae, Lactobacillus salivarius, Lactobacillus lactis, Lactobacillus helveticus, Lactobacillus reuteri, Lactobacillus amylovorus, Lactobacillus crispatus, Lactobacillus curvatus, Lactobacillus delbrueckii subssp. Delbrueckii, Lactobacillus delbrueckii an all its subspecies, Lactobacillus grasseri, Lactobacillus johnsonii, Lactobacillus delbrueckii subsp. bulgaricus, Lactobacillus fermentum, Lactobacillus brevis, Lactococcuc lactis subsp. Lactis, Lactococcus lactis subsp. cremoris, Leuconostoc spp., Enterococcus faecium, Pediococcus pentosaceus, Pedicoccus acidilactici, Bifidobacterium longum, Bifidobacterium breve, Bifidobacterium infantis, Bifidobacterium lactis, Streptococcus thermophiles* and a yeast, preferably a Saccharomyces, more preferably S. *boulardii.*

The probiotic microorganism can be in principle also derived by genetic engineering (designer microorganisms, derived by genetic engineering of an existing microorganism or synthesized by DNA synthesis).

In an additional and/or alternative embodiment, the probiotic microorganism is present in the infant formula in an amount ranging from 10⁶ cfu/g to 10¹¹ cfu/g. Preferably, the probiotic microorganism is present in the infant formula in an amount of at least 10⁶ cfu/g, at least 107 cfu/g, at least 10⁸ cfu/g, at least 10⁹ cfu/g or at least 10¹⁰ cfu/g. The number of microorganisms in the infant formula may vary, but is high enough to colonize the gastrointestinal tract of the infant.

In an additional and/or alternative embodiment, the infant formula further contains at least one prebiotic compound. Said prebiotic is intended to enhance metabolism, growth, multiplication of desired (i.e. beneficial) microbial species in the infant's microbiota and/or the at least one probiotic microorganism within the infant formula. Said at least one prebiotic enhances the modulation of the infant's microbiota towards a desired composition in that the prebiotic faciliates colonization of the gastrintestinal tract by said desired and/or probiotic microorganisms.

In an additional and/or alternative embodiment, the at least one prebiotic is selected from the group consisting of non-digestable oligosaccharides, inulin, fructans, xylooligosaccharides, soyaoligosaccharides, maltose, isomaltosaccharides, fructooligosaccharide (FOS), galactooligosaccharide (GOS), *N*-acetyl-lactosamine, pectin-derived acidic oligosaccharides and milk oligosaccharides (e.g. human milk oligosaccharides (Urashima, T. et al., (2011) Milk Oligosaccharides, Nova Biomedial Books. ISBN 978-1-61122-831-1; Hickey, R. et al (2009) Harnessing milk oligosaccharides for nutraceutical applications. In: Dairy-derived ingredients. Food and neutraceutical uses. Corredig, M. Ed. Woodhead Publishing pp 308-343, ISBN 978-1-84569-465-4; )). The human milk oligosaccharides may be selected from the group consisting of 2'-fucosyllactose, 3-fucosyllactose, 2',3-difucosyllactose, lacto-*N*-triose II, lacto-*N*-tetraose, lacto-*N-*neotetraose, lacto-*N*-fucopentaose I, lacto-*N*-neofucopentaose, lacto-*N-*fucopentaose II, lacto-*N*-fucopentaose III, lacto-*N*-fucopentaose V, lacto-*N-*neofucopentaose V, lacto-*N*-difucohexaose I, lacto-*N*-difucohexaose II, 6'-galactosyllactose, 3'-galactosyllactose, lacto-*N*-hexaose, lacto-*N*-neohexaose, 3'-sialyllactose, 6'-sialyllactose and mono- or disialylated lacto-*N*-tetraoses.

In an additional and/or alternative embodiment, the infant formula is present in liquid form or as a solid preparation in form of a powder, in form of granules, microtablets or tablets.

The infant formula meets the compositional requirements set forth in Regulation (EU) 2016/127 and/or in the Code of Federal Regulations (USA) Title 21 107.100 (nutrient specifications). Representative compositions of infant formulae are specified in Tables 1 and 2 (see also Guo M. (2014) Formulation guidelines for infant formula. In: Human milk biochemistry and infant formula manufacturing technology. Guo M. Ed., Woodhead Publishing. pp 141-171, ISBN 978-1-84569-724-2)

**Table 1: Components of a representative infant formula.**

| | |
|---|---|
| Infant formula: | Skimmed milk |
| | Vegetable oils (palm oil, rapeseed oil, sunflower oil) |
| | Human milk oligosaccharide |
| | Skimmed milk powder |
| | Oil of *Mortierella alpine* |
| | Fish oil |
| | Calcium carbonate |
| | Potassium chloride |
| | Vitamin C |
| | Sodium chloride |
| | Vitamin E |
| | Iron acetate |
| | Zinc sulfate |
| | Niacin |
| | Calciu m-D-panthothenate |
| | Copper sulfate |
| | Vitamin A |
| | Vitamin B1 |
| | Vitamin B6 |
| | Magnesium sulfate |
| | Potassium iodate |
| | Folic acid |
| | Vitamin K |
| | Sodium selenite |
| | Vitamin D |

**Table 2: Composition of a representative infant formula. The final concentration is based on a preparation of 13.5 g of the powder in 90 ml of water.**

| | | per 100 g powder | per 100 ml infant formula |
|---|---|---|---|
| Energy | kJ | 2094-2145 | 283 |
| | kcal | 500-512 | 67-68 |
| Fats, hereof: | 9 | 24.2-26.2 | 3.3-3.5 |
| saturated fatty acids | g | 8.7-9.4 | 1.2-1.3 |
| monounsaturated fatty acids | g | 10.4 | 1.4 |
| polyunsaturated fatty acids | g | 5.5-5.9 | 0.7-0.8 |
| Carbohydrates hereof: | g | 56-58 | 7.4-7.9 |
| | | | |
| Sugars | g | 44-56 | 6-7.4 |
| hereof: | | | |
| Lactose | g | 44-56 | 6-7.4 |
| Protein | g | 11.11-11.85 | 1.5-1.6 |
| Salt | g | 0.47-0.59 | 0.06-0.08 |
| Vitamins | | | |
| Vitamin A | µg | 357-358 | 47.3-48.2 |
| Vitamin D | µg | 7.8 | 1.05 |
| Vitamin E | mg | 8.15 | 1.1 |
| Vitamin K | µg | 43.7-44.4 | 5.9-6.0 |
| Vitamin C | mg | 115-118 | 15-16 |
| Vitamin B1 | mg | 0.51-0.60 | 0.068-0.079 |
| Vitamin B2 | mg | 1.3-1.7 | 0.18-0.23 |
| Niacin | mg | 3.63 | 0.49 |
| Vitamin B6 | µg | 526-600 | 71-81 |
| Folic acid | µg | 160-164 | 21.6-21.7 |
| Vitamin B12 | µg | 1.7-1.9 | 0.23-0.25 |
| Biotin | µg | 22-30 | 3.0-3.9 |
| Panthothenic acid | mg | 4.6-5.4 | 0.62-0.72 |
| Minerals | | | |
| Sodium | mg | 187-236 | 25.3-31.2 |
| Potassium | mg | 673-675 | 88.8-91.2 |
| Chloride | mg | 327-333 | 43.1-44.9 |
| Calcium | mg | 460-504 | 62.1-66.5 |
| Phosphorous | mg | 335-352 | 45.2-46.5 |
| Magnesium | mg | 49.3-56.3 | 6.66-7.43 |
| Iron | mg | 4.15 | 0.56 |
| Zinc | mg | 3.7-3.8 | 0.49-0.51 |
| Copper | µg | 274 | 37 |
| Manganese | µg | 96.3 | 13 |
| Fluoride | µg | 30.4-32.6 | 4.1-4.4 |
| Selenium | µg | 11.1-12.3 | 1.5-1.6 |
| Iodine | µg | 101.5-103.7 | 13.7-14 |

According to the second aspect, the infant formula as disclosed herein before is used for the modulation of the gastrointestinal microbiota of an infant. Using bacteriophages, the number of undesired bacteria can be reduced or even eliminated from a given microbiome. The infant formula is used in that it is fed to an infant in need thereof, i.e. an infant possessing an undesired composition of its microbiota.

According to the third aspect, disclosed in a method for altering or modulating the gastrointestinal microbiota of an infant, wherein an infant formula as disclosed herein before is fed to an infant in need thereof.

In an additional and/or alternative embodiment, the method comprises analyzing the composition of the microbial species that are present in the infant's gastrointestinal microbiome, comparing the results with the composition of microbial species of a reference microbiome, determining a difference in the composition of the microbial species, deciding on the microbial species that have to be targeted by the virus preparation of the infant food, deciding on the lytic virus preparation for targeting the microbial species to be lysed, and selecting an infant formula containing the lytic virus preparation that targets said microbial species.

During the manufacturing process, the virus as well as probiotic cultures are subject to various kinds of stress (e.g. oxygen, heat, cold stress etc.). Most importantly an infant and toddler nutrition product usually possesses shelf lives of several months to several years. Thus, loss of biological activity and cell viability can take place during storage of the virus and/or probiotic strain as an ingredient or as a part of a food product. In particular, the transit of the virus or the probiotic bacteria through the acidic condition of the stomach, or the exposure to the bile salts in the small intestine represent serious issues to the virus activity and strain viability. Especially *Bifidobacterium spp,* struggle to survive these harsh conditions. Several methods have been developed for the save delivery of probiotic bacteria to the gastrointestinal tract. These methods can also be employed for the delivery of viruses and in particular for bacteriophages. In particular encapsulation technologies are widely used for the delivery of probiotic to the gut (Kasipathy K. (2014) Microencapsulation for Gastrointestinal Delivery of probiotic bacteria. In: Nano- and Microencapsulation for Foods. Kwak H.-S. Ed. Wiley Blackwell pp 167-197, ISBN 978-1-118-29233-4; Manoijlovic et al., (2009) Encapsulation of probiotics for us in food products. In: Encapsulation technologies for active food ingredients and food processing. Zuidam, N.J. & Nedovic, V.A. Eds. Springer New York pp 269-302, ISBN 978-1-4419-1007-3). Microencapsulation

The present invention will be described with respect to particular embodiments, but the invention is not limited thereto but only by the claims. Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

### Example 1: Isolation of bacteriophages targeting enteropathogens

The routes of transmission of *Campylobacter jejuni* are unfortunately not well understood. Thus, the best source to isolate a bacteriophage (bacteria virus) are from environments where the phages host is highly prevalent. *Campylobacter* spp. are ubiquitous in temperate environments but favor the intestine of birds, where *Campylobacter* colonizes the gut as a commensal. Therefore, chicken represent a particular good source for *Campylobacter jejuni* specific bacteriophages (Furuta et al., (2017) Characterization and application of lytic bacteriophages against Campylobacter jejuni isolated from poultry in Japan. Biocontrol. Science Vol 22 No. 4 pp213-221; Carvalho et al., (2009) Method for bacteriophage isolation against target Campylobacter strains, Letters in Applied Microbiology 50 (2010) 192-197). In particular, the chickens' gut, due to its low pH (pH 2-4), represents a good source to isolate bacteriophages which are stable at low pH.

Most ideally a bacteriophage is isolated by isolating the Campylobacter strain first from the same location. If not possible a Campylobacter strain from a culture collection (ATCC, DSMZ, NCTC) such as the C. *jejuni* NCTC 12662, *Campylobacter jejuni* DSMZ 104743, *Campylobacter jejuni* DSMZ 24114, *Campylobacter jejuni* DSMZ 24115, *Campylobacter jejuni* DSMZ 24267 can be used.

Campylobacter specific bacteriophages have been isolated from various environmental samples such as poultry, feces and intestines of chickens and ducks, abattoir effluents, human feces, sewage, as well as pig and poultry manure (Grajewski et al., (1985) Development of a bacteriophage typing system for Campylobacter jejuni and Campylobacter coli. J. Clin. Microbiol. 22 pp 13-18. Atterbury et al., (2003) Isolation and characterization of campylobacter bacteriophages from retail poultry. Appl. Environ. Microbiol. 69, pp 4511-4518. Connerton et al., (2004) Longitudinal study of Campylobacter jejuni bacteriophages and their hosts from broiler chickens. Appl. Environ. Microbiol. 70, pp 3877-3883. EI-Shibiny et al., (2005) Enumeration and diversity of campylobacters and bacteriophages isolated during the rearing cycles of free-range and organic chickens. Appl. Environ. Microbiol. 71, pp 1259-1266. Hansen et al., (2007) Characterization of Campylobacter phages including analysis of host range by selected Campylobacter Penner serotypes. BMC Microbiol. 7, 90. Loc Carrillo et al., (2007) Free-range layer chickens as a source of Campylobacter bacteriophage. Antonie Van Leewenhoek 92, pp 275-284. Owens et al., (2012) The isolation and characterization of Campylobacter jejuni bacteriophages from free range and indoor poultry. Vet. Microbiol. 162 (1) pp 144-150.).

A particular useful method for the isolation of Campylobacter phages is the method described by Salama et al., (1989) Improved method for the isolation of Campylobacter jejuni and Campylobacter coli bacteriophages. Lett. Appl. Microbiol. 8 pp 5-7.). According to his method phages are eluded from for example 10 g of soil, feces or intestinal content by suspension of the sample material (1:10) in a suitable buffered solution such as Salt-Magnesium (SM) buffer (SM buffer 0.05 mol l⁻¹, Tris-HCI [pH 7.5], 0.1 mol l⁻¹ NaCl, 0.008 mol ^{l-1} MgSO₄). The suspension is then centrifuges to remove debris and the supernatant transferred to a new tube and centrifuged at higher speeds to remove most bacteria. Finally, the resulting supernatant is sterile filtered using a low-protein binding sterile filter. The sterile filtered filtrate is the used to infect Campylobacter strains (either isolated from the environment, or from clinics (e.g. stool samples from infected patents) or obtained from culture collections such as from the Deutsche Sammlung von Mikroorganismen und Zellkulturen (DMSZ) in Braunschweig, Germany). The filtrate is then applied as 10 µl spots onto lawns, or mixed with 100 µl bacterial culture and poured onto plates. Incubation of all plates and broths are best performed at 37-42°C under micro-aerobic conditions (5% O₂, 5% H₂, 10% CO₂, 80% N₂). The described method is simple and can also be adapted to the isolation of bacteriophages of Enterobacteria such as *Salmonella typhimurium* or *Escherichia coli* specific phages. Any plaques seen are then cored-out using a sterile 1 ml pipette tip and suspended into SM buffer. Single phage plaques are the propagated at least three times to ensure the isolation of a single phage clone.

The proper preparation of the target bacteria strain is also crucial for the isolation of a bacteriophage. In the case of Campylobacter phages, the target Campylobacter strain can be cultured in brain heart infusion broth (BHI, beef heart 5 g/l, calf brains 12.5 g/l, disodium hydrogen phosphate 2.5 g/l glucose 2 g/l, peptone 10 g/l, NaCl 5 g/l (pH 7.4)) supplemented with 10 mM MgSO₄ and 1 mM CaCl₂. The presence of certain cations can be necessary for some phage types to attach to the bacteria cell surface.

The method can be also adapted to Bacteroides spp. or Clostridia spp., however medium and cultivation condition have to be adapted. Bacteroides spp. and Clostridia spp. are best cultivated under strict anaerobic conditions using anaerobia gas (90% N₂, 5% H₂, 5% CO₂). *Clostridium difficile* can be cultured similarly in BHI broth, YT broth (3 % tryptone, 2% yeast extract, pH 7,4) or in Medium 78, 339 and 1203 (see DSMZ media list). For the isolation of *Clostridium difficile* bacteriophages either environmental isolates (e.g. isolated from feces from a symptomatic patient) or strains from culture collections, such as but not limited to C. *difficile* DSM 1296 or DSM 27147, *C*. *difficile* NCTC 13366 and *C*. *difficile* ATCC 43598, can be used.

Besides isolating bacteriophages from natural sources, bacteriophages can also be obtained from culture collection (e.g. DSMZ Braunschweig Germany), or be made synthetically by gene synthesis. For example at DSMZ several phages of *Yersina enterocolitica* are deposited like the Yersina phage Phi80-18 DSM 23253, Yersina phage RhiR1-37 DSM 23251 or Yersina phage RhiR8-01 DSM 23254. Some of the phages are also fully sequence like Yersina phage RhiR8-01 GenBank accession number HE956707.2 or the Yersina phage yhiYe03-12 AJ251805. Also, the Yersina phage yhiYe03-12 is deposited and available from culture collection DSM 23252.

### Example 2: Infant formula containing a lytic bacteriophage preparation

A spray dried and a liquid infant formula as set forth in Table 3 may be prepared:

**Table 3: Composition of an infant formula containing a lytic bacteriophage targeting Campylobacter jejuni. The final concentration is based on a preparation of 13.5 g of the powder in 90 ml of water. * for C. jejuni-specific bacteriophage CP8 see Carrillo et al. (2005) Appl. Environ. Microbiol. 71: 6554 - 6563.**

| | | per 100 g powder | per 100 ml infant formula |
|---|---|---|---|
| Energy | kJ | 2094-2145 | 283 |
| | kcal | 500-512 | 67-68 |
| Fats, hereof: | g | 24.2-26.2 | 3.3-3.5 |
| saturated fatty acids | g | 8.7-9.4 | 1.2-1.3 |
| monounsaturated fatty acids | g | 10.4 | 1.4 |
| polyunsaturated fatty acids | g | 5.5-5.9 | 0.7-0.8 |
| Carbohydrates hereof: | g | 56-58 | 7.4-7.9 |
| | | | |
| Sugars | g | 44-56 | 6-7.4 |
| hereof: | | | |
| Lactose | g | 44-56 | 6-7.4 |
| Protein | g | 11.11-11.85 | 1.5-1.6 |
| Salt | g | 0.47-0.59 | 0.06-0.08 |
| Vitamins | | | |
| Vitamin A | µg | 357-358 | 47.3-48.2 |
| Vitamin D | µg | 7.8 | 1.05 |
| Vitamin E | mg | 8.15 | 1.1 |
| Vitamin K | µg | 43.7-44.4 | 5.9-6.0 |
| Vitamin C | mg | 115-118 | 15-16 |
| Vitamin B1 | mg | 0.51-0.60 | 0.068-0.079 |
| Vitamin B2 | mg | 1.3-1.7 | 0.18-0.23 |
| Niacin | mg | 3.63 | 0.49 |
| Vitamin B6 | µg | 526-600 | 71-81 |
| Folic acid | µg | 160-164 | 21.6-21.7 |
| Vitamin B12 | µg | 1.7-1.9 | 0.23-0.25 |
| Biotin | µg | 22-30 | 3.0-3.9 |
| Panthothenic acid | mg | 4.6-5.4 | 0.62-0.72 |
| Minerals | | | |
| Sodium | mg | 187-236 | 25.3-31.2 |
| Potassium | mg | 673-675 | 88.8-91.2 |
| Chloride | mg | 327-333 | 43.1-44.9 |
| Calcium | mg | 460-504 | 62.1-66.5 |
| Phosphorous | mg | 335-352 | 45.2-46.5 |
| Magnesium | mg | 49.3-56.3 | 6.66-7.43 |
| Iron | mg | 4.15 | 0.56 |
| Zinc | mg | 3.7-3.8 | 0.49-0.51 |
| Copper | µg | 274 | 37 |
| Manganese | µg | 96.3 | 13 |
| Fluoride | µg | 30.4-32.6 | 4.1-4.4 |
| Selenium | µg | 11.1-12.3 | 1.5-1.6 |
| Iodine | µg | 101.5-103.7 | 13.7-14 |
| Bacteriophage CP8* | pfu | 1 x 10⁴ - 1 x 10⁵ | 1.35 x 10³ - 1.35 x 10⁴ |

### Example 3: Infant formula containing a lytic bacteriophage preparation and an HMO

A spray dried and a liquid infant formula as set forth in Table 3 may be prepared:

**Table 4: Composition of an infant formula containing a lytic bacteriophage targeting Campylobacter jejuni. The final concentration is based on a preparation of 13.5 g of the powder in 90 ml of water. * for C. jejuni-specific bacteriophage CP34 see Carrillo et al. (2005) Appl. Environ. Microbiol. 71: 6554 - 6563.**

| | | per 100 g powder | per 100 ml infant formula |
|---|---|---|---|
| Energy | kJ | 2094-2145 | 283 |
| | kcal | 500-512 | 67-68 |
| Fats, hereof: | g | 24.2-26.2 | 3.3-3.5 |
| saturated fatty acids | g | 8.7-9.4 | 1.2-1.3 |
| monounsaturated fatty acids | g | 10.4 | 1.4 |
| polyunsaturated fatty acids | g | 5.5-5.9 | 0.7-0.8 |
| Carbohydrates hereof: | g | 56-58 | 7.4-7.9 |
| | | | |
| Sugars | g | 44-56 | 6-7.4 |
| hereof: | | | |
| Lactose | g | 44-56 | 6-7.4 |
| HMOs | | | |
| hereof | | | |
| 2'-FL | g | 1.85-2.22 | 0.25-0.30 |
| Protein | g | 11.11-11.85 | 1.5-1.6 |
| Salt | g | 0.47-0.59 | 0.06-0.08 |
| Vitamins | | | |
| Vitamin A | µg | 357-358 | 47.3-48.2 |
| Vitamin D | µg | 7.8 | 1.05 |
| Vitamin E | mg | 8.15 | 1.1 |
| Vitamin K | µg | 43.7-44.4 | 5.9-6.0 |
| Vitamin C | mg | 115-118 | 15-16 |
| Vitamin B1 | mg | 0.51-0.60 | 0.068-0.079 |
| Vitamin B2 | mg | 1.3-1.7 | 0.18-0.23 |
| Niacin | mg | 3.63 | 0.49 |
| Vitamin B6 | µg | 526-600 | 71-81 |
| Folic acid | µg | 160-164 | 21.6-21.7 |
| Vitamin B12 | µg | 1.7-1.9 | 0.23-0.25 |
| Biotin | µg | 22-30 | 3.0-3.9 |
| Panthothenic acid | mg | 4.6-5.4 | 0.62-0.72 |
| Minerals | | | |
| Sodium | mg | 187-236 | 25.3-31.2 |
| Potassium | mg | 673-675 | 88.8-91.2 |
| Chloride | mg | 327-333 | 43.1-44.9 |
| Calcium | mg | 460-504 | 62.1-66.5 |
| Phosphorous | mg | 335-352 | 45.2-46.5 |
| Magnesium | mg | 49.3-56.3 | 6.66-7.43 |
| Iron | mg | 4.15 | 0.56 |
| Zinc | mg | 3.7-3.8 | 0.49-0.51 |
| Copper | µg | 274 | 37 |
| Manganese | µg | 96.3 | 13 |
| Fluoride | µg | 30.4-32.6 | 4.1-4.4 |
| Selenium | µg | 11.1-12.3 | 1.5-1.6 |
| Iodine | µg | 101.5-103.7 | 13.7-14 |
| Bacteriophage CP34* | pfu | 1 x 10⁵ | 1.35 x 10⁴ |

### Example 4: Infant formula containing a lytic bacteriophage preparation, HMOs and a probiotic

A spray dried and a liquid infant formula as set forth in Table 5 may be prepared:

**Table 5: Composition of an infant formula containing a lytic bacteriophage targeting Campylobacter jejuni. The final concentration is based on a preparation of 13.5 g of the powder in 90 ml of water. * for C. jejuni-specific bacteriophage CP34 see Carrillo et al. (2005) Appl. Environ. Microbiol. 71: 6554 - 6563.**

| | | per 100 g powder | per 100 ml infant formula |
|---|---|---|---|
| Energy | kJ | 2094-2145 | 283 |
| | kcal | 500-512 | 67-68 |
| Fats, hereof: | g | 24.2-26.2 | 3.3-3.5 |
| saturated fatty acids | g | 8.7-9.4 | 1.2-1.3 |
| monounsaturated fatty acids | g | 10.4 | 1.4 |
| polyunsaturated fatty acids | g | 5.5-5.9 | 0.7-0.8 |
| Carbohydrates hereof: | g | 56-58 | 7.4-7.9 |
| | | | |
| Sugars | g | 44-56 | 6-7.4 |
| hereof: | | | |
| Lactose | g | 44-56 | 6-7.4 |
| HMOs | | | |
| hereof | | | |
| 2'-FL | g | 1.85-2.22 | 0.25-0.30 |
| LN*n*T | mg | 0-111.11 | 0-15 |
| Protein | g | 11.11-11.85 | 1.5-1.6 |
| Salt | g | 0.47-0.59 | 0.06-0.08 |
| Vitamins | | | |
| Vitamin A | µg | 357-358 | 47.3-48.2 |
| Vitamin D | µg | 7.8 | 1.05 |
| Vitamin E | mg | 8.15 | 1.1 |
| Vitamin K | µg | 43.7-44.4 | 5.9-6.0 |
| Vitamin C | mg | 115-118 | 15-16 |
| Vitamin B1 | mg | 0.51-0.60 | 0.068-0.079 |
| Vitamin B2 | mg | 1.3-1.7 | 0.18-0.23 |
| Niacin | mg | 3.63 | 0.49 |
| Vitamin B6 | µg | 526-600 | 71-81 |
| Folic acid | µg | 160-164 | 21.6-21.7 |
| Vitamin B12 | µg | 1.7-1.9 | 0.23-0.25 |
| Biotin | µg | 22-30 | 3.0-3.9 |
| Panthothenic acid | mg | 4.6-5.4 | 0.62-0.72 |
| Minerals | | | |
| Sodium | mg | 187-236 | 25.3-31.2 |
| Potassium | mg | 673-675 | 88.8-91.2 |
| Chloride | mg | 327-333 | 43.1-44.9 |
| Calcium | mg | 460-504 | 62.1-66.5 |
| Phosphorous | mg | 335-352 | 45.2-46.5 |
| Magnesium | mg | 49.3-56.3 | 6.66-7.43 |
| Iron | mg | 4.15 | 0.56 |
| Zinc | mg | 3.7-3.8 | 0.49-0.51 |
| Copper | µg | 274 | 37 |
| Manganese | µg | 96.3 | 13 |
| Fluoride | µg | 30.4-32.6 | 4.1-4.4 |
| Selenium | µg | 11.1-12.3 | 1.5-1.6 |
| Iodine | µg | 101.5-103.7 | 13.7-14 |
| Bacteriophage CP34* | pfu | 1 x 10⁵ | 1.35 x 10⁴ |
| *Lactobacillus reuteri (DSM 17938)* | cfu | 6 x 10⁵ | 8 x 10⁴ |

## Claims

1. An infant formula or toddler nutrition product containing a lytic virus preparation, wherein said virus preparation is lytic to selected microorganisms of an infant's or toddler's microbiota for producing lysis of the selected microorganisms in the infant's or toddler's gastrointestinal tract.

2. The infant or toddler formula or nutrition product according to claim 1, wherein said virus preparation comprises at least one bacteriophage or prophage, said at least one prophage being preferably present as extrachromosomal plasmid form or integrated into the genome of a microorganism.

3. The infant or toddler formula according to claim 1 or 2, wherein said at least one bacteriophage or prophage targets a microorganism selected from the group consisting of gram positive and/or gram negative bacteria, preferably a microorganism selected from one of the families selected from the group consisting of Alcaligenaceae, Bacteroidaceae, Bifidobacteriaceae, Caulobacteraceae, Christenssenellaceae, Clostridiaceae, Coriobacteriaceae, Enterobacteriaceae, Enterococcaceae, Erysipelotrichaceae, Halomonadaceae, Helicobacteraceae, Lachnospiraceae, Peptostreptococcaceae, Phyllobacteriaceae, Porphyromonadaceae, Prevotellaceae, Rikenellaceae, Ruminococcaceae, Streptococcaceae, Veillonellaceae, Vibrionaceae, Verrucomicrobiaceae.

4. The infant or toddler formula or a nutrition product according to any one of claims 1 to 3, wherein said at least one bacteriophage or prophage targets a microorganism selected from one of the genera selected from the group consisting *of Bacillus, Bacteroides, Campylobacter, Cryptococcus, Enterobacter, Enterococcus, Escherichia, Lactobacillus, Haemophilus, Helicobacter, Klebsiella, Listeria, Prevotella, Proteus, Pseudomonas, Salmonella, Shigella and Staphylococcus, Streptococcus, Rothia Staphylococcus, Clostridium, Escherichia, Shigella, Salmonella,* , *Chloerae, Vibrio,* and *Yersinia.*

5. The infant or toddler formula or nutrition product according to any one of claims 1 to 4, wherein said at least one *bacteriophage or prophage targets a microorganism selected from the group consisting of Bacteroidetes bacterium, Bacteroides nordii, Bacteroides fragilis, Caminicella sporogenes, Campylobacter jejuni, Capnocytophaga ochracea, Clostridium difficile, Dysgonomonas gadei, Enterobacter cloacae, Escherichia coli, Eubacterium sp. Helicobacter acinonachis, Helicobacter anseris, Helicobacter aurati, Helicobacter bilis, Helicobacter bizzozeronii, Helicobacter brantae, Helicobacter Canadensis, Helicobacter canis, Helicobacter cetorum, Helicobacter cholecystus, Helicobacter cinaedi, Helicobacter cynogastris, Helicobacter felis, Helicobacter fenelliae, Helicobacter ganmani, Helicbacter hepaticus, Helicobacter mesocricetorum, Helicobacter marmotae, Helicobacter muridarum, Helicobacter mustelae, Helicobacter pametensis, Helicobacter pullorum, Helicobacter pylori, Helicobacter rappini, Helicobacter rodentium, Helicobacter salomonis, Helicobacter trogontum, Helicobacter typhlonius, Helicobacter winghamensis, Nubsella zeaxanthinifaciens, Parabacterioides merdae, Parabacteroides goldsteinii, Pedobacter koreensis, Petrimonas sulfuriphila, Porphyromonas catoniae, Porphyromonase sp., Prevotellacease bacterium, Proteiniphilum acetatigenes, Riemerella nanatipstifer, Salmonella enterica, Salonella bongori, Salmonella subterranean, Salamonella typhimurium, Shigella dysenteriae, Shigella flexneri, Shigella boydii, Shigella sonnei, Spingobacterium sp., Vibrio cholerae and Staphylococcus aureus.*

6. The infant or toddler formula or nutrition product according to any one of claims 1 to 5, wherein the bacteriophage or prophage is selected from the order of Caudavirales; Microviridae, Corticoviridae, Tectiviridae, Leviviridae, Cystoviridae, Inoviridae, Lipothrixviridae, Rudiviridae, Plasmaviridae, Fuselloviridae or Ligamenvirales.

7. The infant or toddler formula or nutrition product according to any one of claims 1 to 6, wherein the virus is present in an amount of between 1 pfu/g and 10²⁰ pfu/g in solid products, or between 0.001 pfu/ml and 10⁹ pfu/ml for liquid products.

8. The infant formula according to any one of claims 1 to 7, wherein said nutritional composition further contains at least one probiotic microorganism.

9. The infant or toddler formula according to claim 8, wherein said at least one probiotic microorganism is selected from the group consisting of *Bacillus coagulans, Lactobacillus acidophilus, Lactobacillus plantarum, Lactobacillus casei subsp. casei, Lactobacillus casei subsp. rhamnosus, Lactobacillus zeae, Lactobacillus salivarius, Lactobacillus lactis, Lactobacillus helveticus, Lactobacillus reuteri, Lactobacillus amylovorus, Lactobacillus crispatus, Lactobacillus curvatus, Lactobacillus delbrueckii subssp. Delbrueckii, Lactobacillus delbrueckii an all its subspecies, Lactobacillus grasseri, Lactobacillus johnsonii, Lactobacillus delbrueckii subsp. bulgaricus, Lactobacillus fermentum, Lactobacillus brevis, Lactococcuc lactis subsp. Lactis, Lactococcus lactis subsp. Cremoris, Leuconostoc spp., Enterococcus faecium, Pediococcus pentosaceus, Pedicoccus acidilactici, Bifidobacterium longum, Bifidobacterium breve, Bifidobacterium infantis, Bifidobacterium lactis, Streptococcus thermophiles* and a yeast, preferably a Saccharomyces, more preferably S. *boulardii.*

10. The infant or toddler formula according to claim 8 or 9, wherein the probiotic microorganism is present in an amount ranging from 10³ cfu/g to 10¹¹ cfu/g.

11. The infant or toddler formula or nutrition product according to any one of claims 1 to 7, wherein said nutritional composition contains at least one prebiotic compound.

12. The infant or toddler formula according to any one of claims 1 to 10, wherein said nutritional composition further contains at least one prebiotic compound.

13. The infant or toddler formula or nutrition product according to claims 11 or 12, wherein said nutritional composition contains at least one prebiotic is selected from the group consisting of non-digestable oligosaccharides, inulin, fructans, xylooligosaccharides, maltose, N-acetyl-lactosamine, fructooligosaccharides (FOS), galactooligosaccharides (GOS) and milk oligosaccharides, preferably human milk oligosaccharides.

14. The infant or toddler formula or nutrition product according to any one of claims 1 to 13, wherein said nutritional composition further contains at least one prebiotic compound and one probiotic microorganism.

15. The infant or toddler formula according to any one of claims 1 to 14, wherein said infant formula or nutrition product is present in liquid form, as powder, in form of granules, microtablets or tablets.

16. A method for altering an infant's gastrointestinal microbiota, the method comprises feeding an infant or toddler formula according to any one of claims 1 to 15.

17. Use of an infant or toddler formula or nutrition product according to any one of claims 1 to 15 for modulating the gastrointestinal microbiota of an infant or toddler.
